# EUROPÄISCHE PATENTANMELDUNG

(11) **EP 1 095 631 A2**
(43) Veröffentlichungstag der Anmeldung: **02.05.2001**
(21) Anmeldenummer: 00250350.6
(22) Anmeldetag: 26.10.2000
(51) Int. Cl.: A61F 2/06

(54) **Stent**

(30) Priorität: 26.10.1999 DE 19951475
(71) Anmelder: BIOTRONIK Mess- und Therapiegeräte GmbH & Co Ingenieurbüro Berlin, 12359 Berlin (DE)
(72) Erfinder: Lootz, Daniel, 18055 Rostock (DE)
(74) Vertreter: Eisenführ, Speiser & Partner

(57) **Zusammenfassung**

Die Erfindung betrifft einen Stent, insbesondere einen Koronarstent, mit mindestens einem ersten rohrförmigen Abschnitt, und mit mindestens einem zweiten rohrförmigen Abschnitt, wobei erster und zweiter rohrförmiger Abschnitt durch mindestens ein erstes Verbindungsmittel miteinander verbunden sind. Die Erfindung zeichnet sich dadurch aus, daß das mindestens eine erste Verbindungsmittel als Doppelsteg ausgebildet ist.

## Beschreibung

Die Erfindung betrifft einen Stent, insbesondere einen Koronarstent, mit mindestens einem ersten rohrförmigen Abschnitt, mit mindestens einem zweiten rohrförmigen Abschnitt, wobei erster und zweiter rohrförmiger Abschnitt durch mindestens ein erstes Verbindungsmittel miteinander verbunden sind.

Derartige Stents sind aus dem Stand der Technik in vielfältiger Weise bekannt. Diese Stents werden u.a. im Zusammenhang mit der perkutanen transluminalen Angioplastie (PCTA, Percutaneous Transluminal Balloon Angioplasty) in der Gefäßchirurgie des Herzens verwendet. Stents können jedoch auch dazu dienen, andere Körperöffnungen aufzuweiten oder aufgeweitet zu halten. Diesem medizinischen Verfahren geht zunächst die Bestimmung des Ortes der Verengung eines Herzkranzgefäßes voraus. Anschließend wird ein sogenannter Angioplastieballon in der Arterie, die die Verengung, die sogenannte Stenose aufweist, an den Ort der Stenose verbracht und dort aufgeblasen. Durch die radial nach außen gerichtete Kraft des aufgeblasenen Ballons wird die Verengung aufgeweitet und im optimalen Fall der ursprüngliche Durchtrittsquerschnitt der zuvor verengten Arterie wieder hergestellt. Neben der erfolgreichen Aufweitung des Gefäßes kann es jedoch zu Nebenwirkungen kommen, die lokale Risse in der Arterie, Zersetzungen und Vorsprünge von Plättchen in das Lumen der Arterie umfassen, so daß es trotz der Aufweitung zu einer Blockade des Gefäßes kommen kann. Darüber hinaus ist es möglich, daß durch elastisches Zurückfedern der Gefäßwand und/oder durch das Wachstum der Intima des Gefäßes erneut eine Stenose auftreten kann. Dies tritt statistisch innerhalb von sechs Monaten bei über 30% der Patienten auf, die mit PCTA behandelt wurden.

Um nun unmittelbar nach der Aufweitung des Gefäßes eine relativ glatte Innenwand des Gefäßes sicherzustellen und eine erneute Stenose vermeiden zu können, wurden die eingangs genannten Stents entwickelt. Diese kleinen Röhrchen dienen u.a. im Zusammenhang mit der PCTA dazu, den durch die Ballonangioplastie erzeugten Gefäßdurchtrittsquerschnitt aufrecht zu erhalten, um somit einen langfristigen Erfolg der PCTA sicherzustellen.

Der Erfolg dieses sogenannten Stenting hängt u.a. auch davon ab, wie gleichförmig sich der Stent an die Gefäßwand anlegen kann. Denn umso gleichförmiger die Gefäßwand abgestützt wird, um so wahrscheinlicher ist es, daß es im Bereich der Stents nicht erneut zu Gefäßverengungen kommt. Dabei bewirkt eine gleichmäßige Stent-Struktur eine relativ glatte Gefäßinnenoberfläche und bei einer relativ glatten Gefäßinnenoberfläche können sich Blutpartikel nur schwer an diese anlagern. Darüber hinaus werden auch Wucherungen der Intima in das Gefäßinnere stärker durch eine gleichmäßige Stentstruktur, die die Gefäßinnenoberfläche relativ geschlossen abgedeckt, verhindert.

Derartige Stents mit einer sogenannten geschlossenen Struktur sind ebenfalls aus dem Stand der Technik bekannt. Beispielhaft sei hier einer der bekanntesten derartiger Stents herausgegriffen, der sogenannte Wallstent. Dieser ist beispielsweise aus dem US 4,655,771 bekannt. Dieser eine geschlossene Struktur aufweisende Stent ist aus zweien gleichmäßig maschenartig gewirkten, in Längsachse des Stents spiralförmig verlaufenden Drähten gebildet.

Der Vorteil der geschlossenen Struktur derartiger Stents wird jedoch durch den Nachteil erkauft, daß diese Stents eine relative longitudinale Steifheit während des Einsetzens aufweisen. Diese Stents erlauben es daher nicht in optimaler Weise, den Stent beim Einführen in Richtung auf die zu behandelnde Stenose durch möglicherweise sehr stark gekrümmte Gefäßabschnitte der Herzkranzarterien zu führen. Auch können diese longitudinal steifen Stents nicht im Bereichh von kurvigen Gefäßabschnitten verwendet werden. Um diese Nachteile einer geschlossenen Struktur zu vermeiden, wurden nunmehr Stents entwickelt, die einen sogenannten modularen Aufbau aufweisen. Bei diesen modularartig aufgebauten Stents sind einzelne, mit einer geschlossenen Struktur versehene Abschnitte, durch flexible Verbindungen miteinander verbunden. Derartige Stents sind beispielsweise aus dem US-Patent 5,104,404 bekannt.

Nachteilig bei den bekannten modularen bzw. segmentierten Stents ist es jedoch, daß es beim Crimpen des Stents (unter Crimpen wird das verschiebesichere Anbringen des Stents auf dem Ballonkatheter durch sanftes radial nach innen gerichtetes Andrücken verstanden) zu einem ungleichmäßigen Verhalten des Stents kommt. Denn die rohrförmigen Abschnitte zeigen beim Crimpen ein anderes Verhalten als die als Verbindungsmittel dienenden einzelnen Stege zwischen den rohrförmigen Abschnitten. Darüber hinaus kommt es nachteiligerweise bei den bekannten Stents aus eben diesen Gründen auch zu einem ungleichmäßigen Aufweitverhalten des Stents. Bei diesem ungleichmäßigen Aufweiten der bekannten Stents kann u.a. ein radiales Abspreizen von einzelnen Stegen auftreten. Dieses Abspreizen ist jedoch unerwünscht, da es die bereits oben erläuterte Gleichmäßigkeit der Innenwandung des aufgeweiteten Stents unterbricht. Darüber hinaus kann es dabei auch zu Verletzungen der Intima des gestenteten Gefäßes kommen. Auch kann es bei herkömmlichen, aus verschiedenen Segmenten zusammengesetzten Stents zu einer sogenannten "hundeknochen"-förmigen Aufweitung des Stents beim Aufweiten desselben kommen. Dabei weiten sich die an den Enden der Stents liegenden rohrförmigen Abschnitte stärker auf als die mehr im Mittenbereich des Stents liegenden rohrförmigen Abschnitte. Auch dies ist aus den oben erläuterten Gründen nachteilig, insbesondere hinsichtlich einer dauerhaften Unterdrückung von weiteren Anlagerungen an die Stentinnenwand.

Aufgabe der vorliegenden Erfindung ist es daher, einen Stent der eingangs genannten Art derart weiter zu bilden, daß die vorgenannten Nachteile vermieden werden, und daß ein Stent zur Verfügung steht, bei dem die Biegeflexibilität segmentierter oder auch als modular bezeichneter Stents erhalten bleibt, während gleichzeitig die Längsstabilität des Stents erhöht wird.

Diese Aufgabe wird bei einem Stent der eingangs genannten Art dadurch gelöst, daß das mindestens eine erste Verbindungsmittel als Doppelsteg ausgebildet ist.

Die Erfindung erreicht mit Hilfe des erfindungsgemäßen Doppelsteges eine gegenüber herkömmlichen segmentierten Stents deutlich erhöhte Längsstabilität des Stents, während gleichzeitig die Biegeflexibilität des modularen Designs des Stents erhalten bleibt. Auf diese Weise ist es Dank der Erfindung möglich, daß der Stent während der Ballondelatation nicht zusammengeschoben wird. Auch können aufgrund der Erfindung die benachbarten rohrförmigen Abschnitte nicht in einen ungewollten Kontakt miteinander geraten. Auf diese Weise ist Dank der Erfindung eine gegenseitige Beeinflussung benachbarter rohrförmiger Abschnitte bei dem Aufweiten des Stents bei der Ballondelatation ausgeschlossen. Darüber hinaus sorgen die erfingsgemäßen Doppelstege im Gegensatz zu den aus dem Stand der Technik bekannten Einzelstegen als Verbindungsmittel der benachbarten rohrförmigen Abschnitte für ein gleichmäßigeres Crimp- und Aufweitverhalten des Stents. Dabei vermeidet die Erfindung vorteilhafterweise u.a. ein radiales Abspreizen von Stegen bei der Ballondelatation und auch eine "hundeknochen"-förmige Aufweitung der rohrförmigen Abschnitte an den Enden des Stents.

Eine besonders bevorzugte Ausführungsform der Erfindung zeichnet sich dadurch aus, daß die rohrförmigen Abschnitte jeweils aus mehreren Zellen bestehen, wobei der erfindungsgemäße Doppelsteg in Längsrichtung des Stents benachbarte Zellen der jeweiligen Abschnitte miteinander verbindet. Durch diesen zellenartigen Aufbau der rohrförmigen Abschnitte wird ein optimales Aufweitverhalten des Stents beim Aufweiten des Stents erreicht.

In einer weiter bevorzugten Ausführungsform sind die Abschnitte aus in Umfangsrichtung benachbart angeordneten, gleichen Zellen aufgebaut. Dies ergibt vorteilhaft ein in Umfangsrichtung gleichmäßiges Crimp- und Aufweitverhalten des Stents.

In einer weiter bevorzugten Ausführungsform sind die Zellen benachbarter Abschnitte gleich aufgebaut. Dabei ist es bevorzugt, wenn alle Zellen aller Abschnitte gleich aufgebaut sind, wobei es insbesondere weiter bevorzugt ist, wenn auch alle Abschnitte identisch aufgebaut sind. Auf diese Weise ergibt sich bei dieser Ausführungsform ein völlig gleichmäßiges Crimp- und Aufweitverhalten des erfingsgemäßen Stents über die gesamte Länge des Stents.

Bei einer besonders bevorzugten Ausführungsform der vorliegenden Erfindung sind die Zellen der Abschnitte aus jeweils 2 einander zugewandten, in Umfangsrichtung der Abschnitte mäandrisch verlaufenden, geschlossenen Stegen aufgebaut, welche Stege zur Bildung der Zellen mittels als Einfachstege ausgebildeter zweiter Verbindungsmittel miteinander verbunden sind. Derartige geschlossene Stege verwirklichen die erfindungsgemäße Aufgabe der Erhaltung der Biegeflexibilität bei gleichzeitiger Längsstabilität des Stents in besonders vorteilhafter Weise, da sich bei dieser Ausführungsform die erfindungsgemäßen Doppelstege zwischen den mäandrischen Stegen erstrecken können, in dem sie je nach Anforderung an den Stent an den oberen oder unteren Scheitelpunkten des in Umfangsrichtung verlaufenden Stegmäanders angreifen. Gleichzeitig wird durch die mäanderförmige Begrenzung der rohrförmigen Abschnitte eine optimal gleichmäßige Aufweitung des Stents möglich.

Bei einer weiter bevorzugten Ausführungsform der Erfindung sind die mäandrischen Stege spiegelsymmetrisch zueinander auf dem Umfang angeordnet. Auf diese Weise werden insbesondere durch die bezüglich der Spiegelebene nach außen stehenden Bögen der mäanderförmigen Stege zur Bildung der Zellen der rohrförmigen Abschnitte herangezogen.

Dabei ist es weiter bevorzugt, wenn die Mäanderform der mäandrischen Stege im wesentlichen dem Verlauf einer Sinuskurve entlang einer Umfangslinie der rohrförmigen Abschnitte entspricht. Auf diese Weise wird ein besonders harmonisches Expansionsverhalten des erfindungsgemäßen Stents sichergestellt, da ein sinusförmig gebogener Steg sich besonders gleichmäßig bei der Expansion des Stents ausdehnt.

Bei einer weiter bevorzugten Ausführungsform der Erfindung ist jeder zweite Bogen der in Umfangsrichtung der rohrförmigen Abschnitte sinusförmig mäandrisch ausgebildeten Stege flacher als die verbleibenden Bögen ausgebildet. Dadurch weisen auch die durch die mäandrischen Stege eingeschlossenen Zellen der rohrförmigen Abschnitte alternierend eine abwechselnde Größe auf. Durch diese Ausführungsform wird erreicht, daß der erfindungsgemäße Stent auch beim Einführen in gebogene Gefäße einen genügend großen Spielraum zwischen den rohrförmigen Abschnitten aufweist. Dieser Spielraum oder Bewegungsfreiraum zwischen den rohrförmigen Abschnitten, der durch die flacher ausgebildeten Bögen der Stege erzeugt wird, stellt somit sicher, daß sich benachbarte rohrförmige Abschnitte beim Biegen des Stents nicht behindern oder ineinander verhaken. Bei dieser Ausführungsform ist es weiter bevorzugt, wenn der oder die Doppelstege die rohrförmigen Abschnitte an Stellen verbindet, an denen die flacheren Bögen der mäandrischen Stege vorgesehen sind. Auf diese Weise wird dem Stent beim Biegen desselben ein besonders großer Spielraum zwischen den rohrförmigen Abschnitten zur Verfügung gestellt, so daß die vorgenannten Vorteile weiter verstärkt werden.

Bei einer weiter besonders bevorzugten Ausführungsform der Erfindung sind die Scheitelpunkte der im wesentlichen sinusförmigen Bögen der mäandrischen Stege in longitudinaler Richtung der rohrförmigen Abschnitte abgeflacht ausgebildet. Bei dieser Ausführungsform sind also die Umkehrpunkte der Mäander mit doppelten Radien versehen. Auf diese Weise wird bei dieser Ausführungsform vorteilhaft ein "eselsohren"-artiges Abspreizen der Bögen der Mäander vermieden. Dadurch wiederum wird vorteilhaft eine mit einem solchen Abspreizen verbundene Verletzung der Gefäßintima verhindert. Insoweit wird auch bei dieser Ausführungsform insgesamt ein gleichmäßigeres Crimp- und Aufweitverhalten des Stents erzielt. Darüber hinaus werden durch die abgeflachten Scheitelpunkte bzw. durch die Doppelradien der Mäanderumkehrpunkte die mechanischen Belastungen auf eine bevorzugte auf der Stentoberfläche, das heißt auf den Stegen vorgesehene Beschichtung verringert. Denn durch die Doppelradien wird beim Aufweiten des Stents die Belastung auf die Beschichtung bzw. die Belastung auf die Haftung der Beschichtung an der Stegoberfläche nicht nur auf einen Umkehrpunkt im Mäander ausgeübt, sondern auf die beiden durch die Doppelradien gebildeten Teilbereiche der Umkehr- bzw. Scheitelpunkte der Mäander. Durch diese Verteilung der Deformation der Mäander beim Aufweiten des Stents auf die beiden Radien-Teilbereiche der Mäanderbögen wird somit insgesamt die Belastung der Beschichtung eines Stents vorteilhaft verringert.

Eine weitere bevorzugte Ausführungsform der Erfindung zeichnet sich dadurch aus, daß die zweiten Verbindungsmittel jeweils in longitudinaler Richtung der rohrförmigen Abschnitte benachbart angeordnete Bereiche der mäandrischen Stege verbinden. Dabei ist es weiter bevorzugt, wenn die zweiten Verbindungsmittel zwei mäandrische Stege jeweils in der Nähe ihrer longitudinal maximal beabstandeten Bereiche verbinden. Dabei verlaufen die zweiten als Einfachstege ausgebildeten Verbindungsmittel bevorzugt im wesentlichen in longitudinaler Richtung der rohrförmigen Abschnitte. Somit wird bei dieser Ausführungsform vorteilhafterweise eine Minimierung der Längskontraktion des Stents beim Aufweiten desselben erzielt. Auch dienen diese Längsstege der Längsstabilisierung des Stents. Dabei wirken diese Längsstege bei der Längsstabilisierung des Stents mit den erfindungsgemäßen Doppelstege vorteilhaft zusammen.

Bei einer weiteren besonders bevorzugten Ausführungsform verlaufen die Enden der zweiten als Einfachstege ausgebildeten Verbindungsmittel im wesentlichen in Umfangsrichtung der rohrförmigen Abschnitte. Dabei können sich die Einfachstege bei einer weiter bevorzugten Ausführungsform alternativ dazu oder ergänzend dazu zu ihrem jeweiligen Verbindungspunkt mit den mäandrischen Stegen hin verjüngen. Auf diese Weise bilden dann die als zweite Verbindungsmittel dienenden Einfachstege zwischen den mäandrischen Stegen Fließgelenke. Diese Fließgelenke stellen eine möglichst geringe Beeinflussung des radialen Aufweitverhalten des Stents beim Aufweiten desselben vorteilhaft sicher.

Bei einer weiteren vorteilhaften Fortbildung der vorliegenden Erfindung sind die Enden der Doppelstege an Stellen der mäandrischen Stege angebracht, in deren unmittelbarer Umgebung auch der entlang des jeweiligen rohrförmigen Abschnittes longitudinal benachbarte Einfachsteg des zweiten Verbindungsmittels befestigt ist. Auf diese Weise wird bei dieser Ausführungsform eine maximal mögliche Längsstabilisierung des Stents erreicht, in dem beide in Längsrichtung des Stents verlaufenden Stege, d.h. der Doppelsteg und der Einfachsteg im wesentlichen miteinander fluchtend in Längsrichtung verlaufen.

Weitere bevorzugte Ausführungsformen der Erfindung sind in den Unteransprüchen angebeben.

Zwei vorteilhafte Ausführungsformen der Erfindung werden nunmehr mit Bezug auf die begleitende Zeichnung erläutert. Die Figuren der Zeichnung zeigen:
- Figur 1: zeigt einen anhand der Abwicklung seiner Mantelfläche dargestellten erfindungsgemäßen Stent.
- Figur 2: zeigt schematisch eine zweite Ausführungsform eines erfindungsgemäßen Stents anhand der Abwicklung der Mantelfläche; und
- Figur 3: zeigt die Ausführungsform der Figur 2 bei gebogenem Stent.

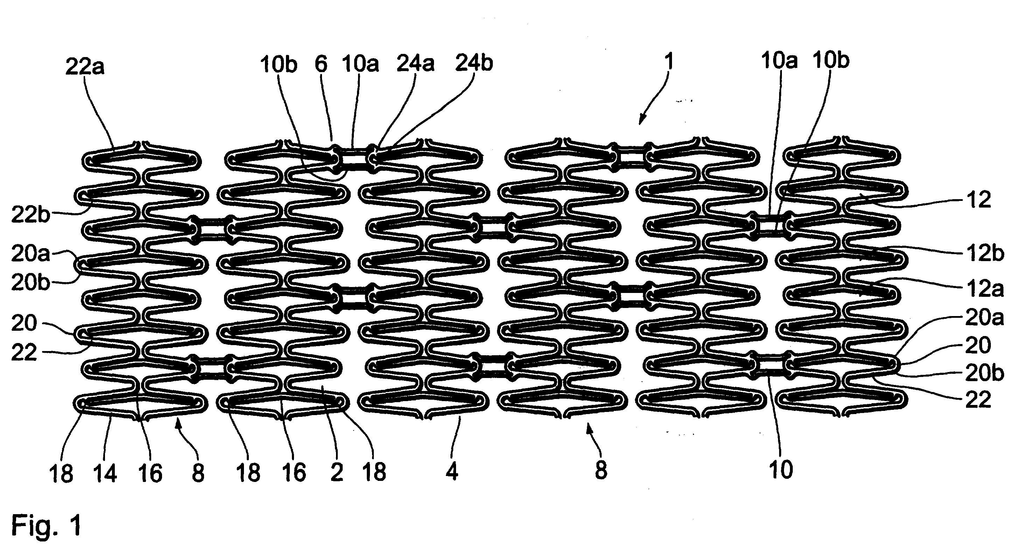

Die Figur 1 zeigt einen erfindungsgemäßen Stent 1. Der Stent 1 ist in der Figur 1 als Abwicklung der Mantelfläche 2 des Stents 1 dargestellt. Im funktionsfähigen Zustand des Stents 1 ist die Mantelfläche 2 mit ihrer in der Figur 1 unten dargestellten Seite 4 mit der in der Figur 1 oben dargestellten Seite 6 verbunden, so daß sich somit der funktionsfähige Stent 1 ergibt.

Die Mantelfläche 2 setzt sich aus 6 rohrförmigen Abschnitten 8 zusammen. Diese rohrförmigen Abschnitte 8 sind natürlich in der in der Figur 1 dargestellten Abwicklung der Mantelfläche 2 nicht rohrförmig. Die rohrförmige Gestalt der rohrförmigen Abschnitte 8 wird erst durch das oben aneinanderfügen der Seiten 4 und 6 der Mantelfläche 2 erreicht.

In longitudinaler Richtung, die in der Figur 1 in der Zeichnungsebene von links nach rechts verläuft, sind die rohrförmigen Abschnitte 8 durch als erfindungsgemäße Verbindungsmittel dienende Doppelstege 10 miteinander verbunden.

Weiterhin sind die rohrförmigen Abschnitte 8 aus mehreren in Umfangsrichtung des Stents 1 benachbart angeordneten Zellen 12 aufgebaut. Die Zellen 12 der rohrförmigen Abschnitte 8 weisen alternierend eine unterschiedliche Größe auf. Im gesamten Stent 1 bzw. in der Mantelfläche 2 des Stents 1 sind jedoch nur zwei unterschiedlich große Zellen 12 vorgesehen. Es handelt sich dabei um die kleineren Zellen 12a und um die größeren Zellen 12b.

Alle Abschnitte 8 sind untereinander identisch ausgebildet, weisen also die gleiche Abfolge von kleineren 12a und größeren 12b Zellen 12 auf.

Die Zellen 12 sind in jedem rohrförmigen Abschnitt 8 jeweils durch zwei einander zugewandte, in Umfangsrichtung der rohrförmigen Abschnitte 8 mäandrisch verlaufende, geschlossene Stege 14 aufgebaut. Die Stege 14 sind wiederum untereinander paarweise mittels im wesentlichen in longitudinaler Richtung verlaufender Einfachstege 16, die als erfindungsgemäße zweite Verbindungsmittel dienen, miteinander verbunden. Diese Einfachstege 16 sind in ihren Mitten dicker ausgebildet als an ihren Enden 18. Die Stege 16 verjüngen sich also zu ihren Endn 18 hin. Weiterhin sind die Enden 18 der Stege 16 im wesentlichen in Umfangsrichtung in Richtung auf die oben in der Figur 1 dargestellte Seite 6 der Mantelfläche 2 gebogen, bevor sie dann an den Stegen 14 befestigt sind.

Die mäanderförmigen Stege 14 weisen einen im wesentlichen sinusförmig verlaufendes Mäander auf. Dabei sind jedoch die jeweiligen Scheitelpunkte 20 der Bögen 22 der Stege 14 abgeflacht, d.h. mit doppelten Radien versehen. Es ergeben sich somit zwei Teilbereiche 20a und 20b, die jeweils die Teilradien der Bögen 22 bilden. Die Einzelstege 16, die die Stege 14 miteinander verbinden, sind alle im Bereich der Teilradien 20a der Bögen 22 angebracht.

Die sinusartige Mäanderform der Stege 14 verläuft alternierend unregelmäßig, in dem große Bögen 22b und kleine Bögen 22a vorgesehen sind. Auf diese Weise werden durch die kleinen Bögen 22a die kleinen Zellen 12a gebildet und durch die großen Bögen 22b die großen Zellen 12b der rohrförmigen Abschnitte 8 gebildet.

Die erfindungsgemäßen Doppelstege 10 sind aus zwei im wesentlichen parallel verlaufenden Einzelstegen 10a und 10b gebildet. Die Doppelstege 10 verbinden die rohrförmigen Abschnitte 8 jeweils immer an benachbart zueinander liegenden kleinen Zellen 12a. Jeweils zwei rohrförmige Abschnitte 8 sind mit jeweils 2 Doppelstegen 10 miteinander verbunden. Dabei sind die Doppelstege 10 so angeordnet, daß zwischen ihnen drei Zellenpaare von rohrförmigen Abschnitten 8 zu liegen kommen. Weiterhin sind die Doppelstege 10 derart versetzt zueinander angeordnet, daß das neben einem ersten Paar liegende zweite Paar rohrförmiger Abschnitte durch die Doppelstege 10 an Stellen miteinander verbunden wirkt, die derart zu den Verbindungsstellen des ersten Paares rohrförmiger Abschnitte 8 versetzt angeordnet sind, daß zwischen beiden Verbindungsstellen jeweils ein Paar von großen Zellen 12b zu liegen kommt.

Die Einzelstege 10a und 10b der Doppelstege 10 sind an ihren Enden zunächst in Richtung einer ersten Diagonalen der Mantelfläche 2 von ihren Befestigungspunkten 24a bzw. 24b weggebogen, um dann direkt in unmittelbarer Nachbarschaft der Befestigungspunkte 24a und 24b an den Stegen 14 zu den Stegen 14 in Richtung der zweiten Diagonalen der Mantelfläche 2 hingebogen zu sein. Die Befestigungspunkte 24a und 24b befinden sich jeweils im Bereich der Teilradien 20a und 20b der kleineren Bögen 22a. Weiterhin sind die Befestigungspunkte 24a der in der Figur 1 oben dargestellten Einzelstege 10a der Doppelstege 10 in unmittelbarer Nähe der in den entsprechenden Zellen 12 angeordneten Einzelstege 16 angeordnet.

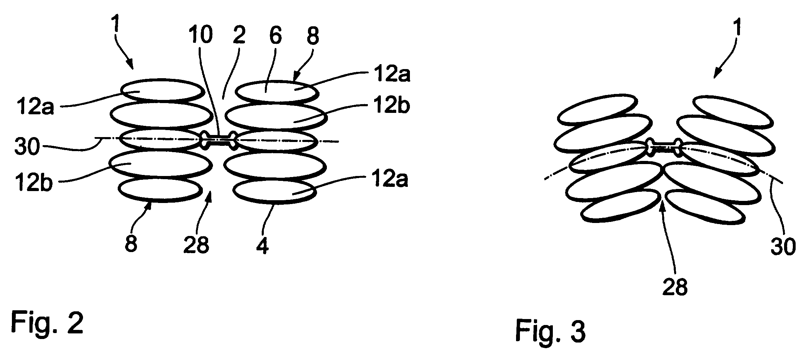

Figur 2 zeigt eine schematische Ansicht der Mantelfläche 2 einer zweiten Ausführungsform des erfindungsgemäßen Stents 1. Teile, die denen der Figur 1 entsprechen, sind mit gleichen Bezugszeichen bezeichnet.

Zwei schematisch dargestellte rohrförmige Abschnitte 8 sind durch einen schematisch dargestellten Doppelsteg 10 miteinander verbunden. Die rohrförmigen Abschnitte 8 weisen kleine Zellen 12a und große Zellen 12b auf. Zwischen den an den Seiten 6 bzw. 4 der Mantelfläche 2 außen liegenden kleinen Zellen 12a der rohrförmigen Abschnitte 8 entsteht durch die Wahl der Länge des Doppelsteges 10 und die Wahl der in Richtung der longitudinalen 30 verlaufenden Erstreckung der Zellen 12a bzw. 12b ein Freiraum 28.

Die Figur 3 zeigt die Ausführungsform der Figur 2 in einem Zustand, bei dem der Stent 1 gebogen ist. Diese Biegung des Stents 1 ist durch die gebogen dargestellte strichpunktierte longitudinale Linie 30 schematisch angedeutet. Die Figur 3 zeigt, daß der Bewegungsspielraum 28 bei der in der Figur 3 dargestellten Biegebewegung nahezu vollständig ausgenutzt wird.

## Patentansprüche

1. Stent, insbesondere Koronarstent (1), mit mindestens einem ersten rohrförmigen Abschnitt (8), mit mindestens einem zweiten rohrförmigen Abschnitt (8), wobei erster und zweiter rohrförmiger Abschnitt (8) durch mindestens ein erstes Verbindungsmittel (10) miteinander verbunden sind, dadurch gekennzeichnet, daß das mindestens eine erste Verbindungsmittel als Doppelsteg (10) ausgebildet ist.

2. Stent nach Anspruch 1, bei dem die rohrförmigen Abschnitte (8) jeweils aus mehreren Zellen (12, 12a, 12b) bestehen, wobei der Doppelsteg (10) in longitudinaler Richtung (30) des Stents (1) benachbarte Zellen (12, 12a, 12b) der Abschnitte (8) miteinander verbindet.

3. Stent nach Anspruch 2, wobei die Abschnitte (8) aus in Umfangsrichtung benachbart angeordneten, gleichen Zellen (12, 12a, 12b) aufgebaut sind.

4. Stent nach einem der Ansprüche 2 oder 3, wobei die Zellen (12, 12a, 12b) benachbarter Abschnitte (8) gleich aufgebaut sind.

5. Stent nach einem der Ansprüche 2 bis 4, wobei alle Zellen (12, 12a, 12b) aller Abschnitte (8) gleich aufgebaut sind.

6. Stent nach einem der Ansprüche 2 bis 5, wobei alle Abschnitte (8) gleich aufgebaut sind.

7. Stent nach einem der Ansprüche 2 bis 6, wobei die Zellen (12, 12a, 12b) der Abschnitte (8) aus jeweils zwei einander zugewandten, in Umfangsrichtung der Abschnitte (8) mäandrisch verlaufenden, geschlossenen Stegen (14) aufgebaut sind, welche Stege (14) zur Bildung der Zellen (8) mittels als Einfachstege (16) ausgebildeter zweiter Verbindungsmittel miteinander verbunden sind.

8. Stent nach Anspruch 7, wobei die mäandrischen Stege (14) bezüglich einer Umfangslinie des durch sie gebildeten Abschnittes (8) spiegelsymmetrisch aufgebaut sind.

9. Stent nach einem der Ansprüche 7 oder 8, wobei die Mäanderform der mäandrischen Stege (14) im wesentlichen dem Verlauf einer Sinuskurve entspricht.

10. Stent nach Anspruch 9, wobei jeder zweite Bogen (22) der in Umfangsrichtung der rohrförmigen Abschnitte (8) sinusförmig mäandrischen Stege (14) flacher als die restlichen Bögen (22) ausgebildet ist.

11. Stent nach Anspruch 10, wobei der oder die Doppelstege (10) mindestens einen der flacheren Bögen (22a) der mäandrischen Stege (14) miteinander verbindet oder verbinden.

12. Stent nach einem der Ansprüche 9 bis 11, wobei die Scheitelpunkte (20) der im wesentlichen sinusförmigen Bögen (22, 22a, 22b) der mäandrischen Stege (14) in longitudinaler Richtung (30) der rohrförmigen Abschnitte (8) abgeflacht ausgebildet sind.

13. Stent nach Anspruch 8 und nach einem der vorstehenden Ansprüche, wobei die zweiten Verbindungsmittel (16) jeweils in longitudinaler Richtung (30) der rohrförmigen Abschnitte (8) benachbart angeordnete Bereiche der mäandrischen Stege (14) verbinden.

14. Stent nach Anspruch 9 und nach einem der vorstehenden Ansprüche, wobei die zweiten Verbindungsmittel (16) zwei mäandrische Stege (14) jeweils in der Nähe ihrer in longitudinaler Richtung (30) der rohrförmigen Abschnitte (8) maximal beabstandeten Bereiche verbinden.

15. Stent nach einem der Ansprüche 7 bis 10 und nach einem der vorstehenden Ansprüche, wobei die zweiten als Einfachstege (16) ausgebildeten Verbindungsmittel im wesentlichen in longitudinaler Richtung (30) der rohrförmigen Abschnitte (8) verlaufen.

16. Stent nach Anspruch 11 und nach einem der vorstehenden Ansprüche, wobei die Enden (18) der zweiten als Einfachstege ausgebildeten Verbindungsmittel (16) im wesentlichen in Umfangsrichtung der rohrförmigen Abschnitte (8) verlaufen.

17. Stent nach einem der Ansprüche 13 bis 16, wobei die zweiten Verbindungsmittel (16) die mäandrischen Stege (14) an Punkten (24a, 24b) minimaler Deformation der mäandrischen Stege (14) bei Expansion des Stents (1) verbinden.

18. Stent nach einem der Ansprüche 13 bis 17, wobei sich die als Einfachstege (16) ausgebildeten zweiten Verbindungsmittel zu Ihrem jeweiligen Verbindungspunkt (24a, 24b) mit den mäandrischen Stegen (14) hin verjüngen.

19. Stent nach einem der vorstehenden Ansprüche, wobei die Stege (10a, 10b) des Doppelsteges (10) im wesentlichen in longitudinaler Richtung (30) der rohrförmigen Abschnitte (8) verläuft.

20. Stent nach Anspruch 19, wobei die Enden der Doppelstege (10) im wesentlichen in Umfangsrichtung der rohrförmigen Abschnitte (8) verlaufen.

21. Stent nach einem der vorstehenden Ansprüche, wobei die Stege (10a, 10b) des Doppelsteges (10) bezüglich einer longitudinal auf dem Stent (1) verlaufenden Linie (30) spiegelsymmetrisch ausgebildet sind.

22. Stent nach einem der vorstehenden Ansprüche, wobei der Doppelsteg (10) oder die Doppelstege (10) die Abschnitte in der Nähe von Stellen (20) des geringsten gegenseitigen longitudinalen Abstandes der Abschnitte miteinander verbindet oder verbinden.

23. Stent nach einem der Ansprüche 7, 8, 13 bis 16 und nach einem der vorstehenden Ansprüche, wobei einer der Stege (10a) des Doppelsteges (10) an einer Stelle (24a) an den mäandrischen Stegen (14) angebracht ist, in deren Nähe auch der entlang des jeweiligen rohrförmigen Abschnittes (8) longitudinal benachbarte Einfachsteg (16) des zweiten Verbindungsmittels an diesem mäandrischen Steg (14) befestigt ist.
